# FASCICULE DE BREVET EUROPEEN

(11) **EP 2 018 838 B1**
(45) Date de publication et mention de la délivrance du brevet: **20.11.2013**
(21) Numéro de dépôt: 08159549.8
(22) Date de dépôt: 02.07.2008
(51) Int. Cl.: A61K 8/06, A61K 8/81, A61Q 19/00, A61K 8/37, A61K 8/86

(54) **Emulsion huile-dans-eau contenant un polymère amphiphile**
Öl-in-Wasser-Emulsion, die ein amphiphiles Polymer enthält
Oil-in-water emulsion containing an amphiphile polymer

(30) Priorité: 20.07.2007 FR 0756636
(43) Date de publication de la demande: 28.01.2009
(73) Titulaire: L'Oréal, 75008 Paris (FR)
(72) Inventeur: L'Alloret, Florence, 75014, Paris (FR); Bressy, Lydie, 69100 Villeurbanne (FR)
(74) Mandataire: Duvert, Sandra

(56) Documents cités:
- EP-A- 1 069 142
- EP-A- 1 302 190
- FR-A- 2 843 695
- FR-A- 2 853 527
- FR-A- 2 853 544

## Description

La présente demande se rapporte à une composition sous forme d'émulsion huile-dans-eau, contenant au moins un polymère amphiphile particulier et un émulsionnant non ionique particulier, et à l'utilisation de la dite composition, notamment pour le soin, le démaquillage et/ou le nettoyage de la peau du corps ou du visage, des cheveux, des lèvres et/ou des yeux.

Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme phase externe, une phase aqueuse, ce qui leur confère, lors de l'application sur la peau, un toucher plus frais, moins gras et plus léger que les émulsions E/H.

Les émulsions H/E classiques sont en général stabilisées par des molécules amphiphiles de faible masse molaire (< 5 000 g/mole) tels que les tensioactifs émulsionnants de type alkylglycérolé ou alkylpolyoxyéthyléné. Toutefois, ces tensioactifs quand ils sont en une certaine quantité, présentent l'inconvénient d'induire un toucher cireux et lourd. En outre, on cherche à disposer de compositions contenant le moins possible de tensioactifs car ils sont parfois mal supportés par les peaux sensibles.

Par ailleurs, il est connu de remplacer les tensioactifs par des polymères carboxyvinyliques modifiés, c'est-à-dire comportant une partie hydrophobe constituée d'une chaîne grasse, tels que les copolymères d'alkyl-C₁₀-C₃₀-acrylate et d'acide acrylique ou méthacrylique, comme les produits commercialisés sous la dénomination PEMULEN par la société Noveon. Toutefois, ces polymères conduisent à des émulsions comprenant des gouttes de grande taille (≈10 - 15 µm), qui sont difficiles à stabiliser dans le cas où l'on veut obtenir des textures fluides, ce qui entraîne un phénomène de crémage de l'émulsion. Ces copolymères sont par ailleurs sensibles au pH, et ils doivent être formulés à un pH supérieur à 6 pour bénéficier de leurs propriétés gélifiantes et émulsionnantes.

En outre, il est connu par le document EP-A-1,069,142 d'utiliser des polymères amphiphiles dérivés d'acide 2-acrylamido 2-méthylpropane sulfonique (AMPS), ces polymères pouvant être utilisés comme épaississant, émulsionnant, dispersant et agent de suspension, notamment dans le domaine cosmétique. Toutefois, les polymères exemplifiés dans ce document ne permettent pas d'obtenir des émulsions H/E qui à la fois aient des propriétés cosmétiques agréables pour l'utilisateur et soient très stables et de réalisation aisée.

La demande EP-1,302,190 décrit des émulsions démaquillantes stabilisées par un polymère amphiphile à base d'AMPS ; l'exemple 1 correspond à une émulsion comprenant 1% de polymère émulsionnant et 20% de phase grasse démaquillante. Lors de l'application sur la peau, l'émulsion se casse et conduit à un dépôt non homogène, correspondant aux textures dites « quick break ». Bien que très intéressant pour le démaquillage, ce comportement ne favorise pas un dépôt homogène d'actifs à la surface de la peau et ne conduit pas à des textures lisses et fondantes à la surface de la peau.

Le document FR-2,843,695 décrit des émulsions H/E contenant un polymère amphiphile d'AMPS non réticulé et un taux d'huiles supérieur à 40 % en poids. Toutefois, une telle quantité de phase huileuse entraîne un effet gras et brillant lors de l'application sur la peau, ce qui peut être rédhibitoire pour l'utilisateur.

Par ailleurs, le document FR-2,853,527 est relatif à des émulsions H/E contenant un polymère amphiphile à base d'AMPS, non réticulé, portant des chaînes alkyles en C₆-C₁₅ et un émulsionnant lipophile et il décrit des compositions dont la texture reste le plus souvent fluide. Toutefois, ces polymères ne permettent d'obtenir des textures plus consistantes telles que des crèmes qu'en utilisant une quantité importante de phase huileuse ou encore en introduisant un gélifiant hydrophile supplémentaire dans la phase aqueuse. Par ailleurs, dans les exemples, la quantité de polymère est d'au moins 0,8%.

Le document FR-2,853,544 décrit des émulsions H/E contenant un polymère amphiphile à base d'AMPS, non réticulé, comportant des proportions spécifiques de parties hydrophobe et hydrophile selon le nombre de carbone de la chaîne alkyle. Ces émulsions se présentent sous forme de textures fluides, et elles contiennent une quantité importante de polymère. L'exemple comparatif 2 de cette demande comprend une teneur élevée en polymère (1,75%), ce qui conduit certes à une émulsion stable sans ajout de tensioactif, mais sa texture est gélifiée et tremblante, d'aspect peu cosmétique et ayant une prise au doigt difficile.

Ainsi, il subsiste le besoin de réaliser des émulsions H/E surmontant les difficultés de l'art antérieur, c'est-à-dire ayant de bonnes qualités cosmétiques (toucher agréable lors de l'application), une bonne stabilité et des textures très variées allant du fluide pulvérisable à la crème consistante, tout en ayant des quantités limitées de phase huileuse, de polymère et de tensioactifs.

La demanderesse a découvert de façon inattendue que l'association de polymères amphiphiles d'AMPS non réticulés particuliers et d'émulsionnants non ioniques en des proportions spécifiques permettait de réaliser de telles émulsions. On peut ainsi obtenir des émulsions huile-dans-eau, stables, possédant de grandes qualités sensorielles avec un toucher glissant et non collant, et pouvant se présenter aussi bien sous forme de produits fluides que sous forme de crèmes épaisses.

Ainsi, la présente invention concerne une composition pour application topique, sous forme d'émulsion huile-dans-eau comprenant une phase huileuse dispersée dans une phase aqueuse, caractérisée en ce qu'elle contient (a) un ou plusieurs émulsionnants non ioniques ayant un point de fusion inférieur à 45°C choisis parmi les esters d'acide gras et de polyol, et (b) un ou plusieurs polymères non réticulés comprenant de (A) 80 à 99 % en moles de motif acide 2-acrylamido 2-méthylpropane sulfonique de formule (I): 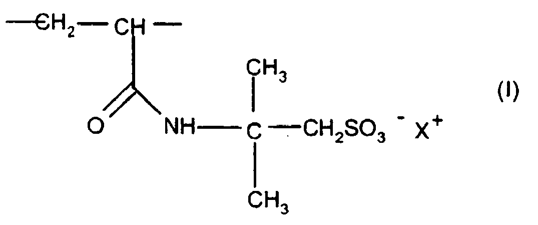 dans laquelle X⁺ est un proton, un cation de métal alcalin, un cation alcalino-terreux ou l'ion ammonium;
et (B) 1 à 20 % en moles de motif de formule (II) suivante : 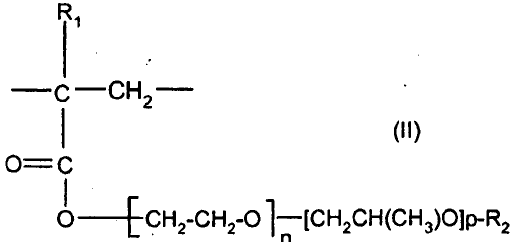 dans laquelle p = o; n est compris entre 6 et 25; R₁ désigne un radical méthyle et R₂ désigne un radical alkyle linéaire ou ramifié comportant de 16 à 25 atomes de carbone,
la quantité de polymère(s) (b) étant inférieure ou égale à 0,6 % en poids par rapport au poids total de la composition, et le rapport en poids entre la quantité de polymère(s) (b) et la quantité d'émulsionnant(s) (a) allant de 0,7 à 1,3,
et en ce que la phase huileuse est présente en une quantité inférieure à 40 % en poids par rapport au poids total de la composition.

Dans la présente invention, la quantité de phase huileuse n'inclut pas la quantité d'émulsionnants non ioniques utilisés selon l'invention.

La proportion en moles de monomère hydrophobe de formule (II) correspond au pourcentage molaire de chaîne latérale de motif (II) par rapport à la somme des motifs (1) et (II).

On entend ici par « application topique », une application externe sur les matières kératiniques, que sont notamment la peau, le cuir chevelu, les cils, les sourcils, les ongles, les muqueuses et les cheveux.

La composition selon l'invention étant destinée à une application topique, elle contient un milieu physiologiquement acceptable, c'est-à-dire compatible avec la peau, les muqueuses, les cheveux et le cuir chevelu.

La composition selon l'invention présente l'avantage d'avoir une bonne innocuité et de bonnes propriétés cosmétiques, c'est-à-dire une texture homogène et agréable à l'application. En outre, elle est bien stable dans le temps. Par "stable", on entend une composition qui, après au moins un mois de stockage ou plus, à toutes températures comprises entre 4°C et 45°C, ne présente ni changement macroscopique de couleur, d'odeur ou de viscosité, ni variation de pH, ni changement d'apparence microscopique.

Les polymères utilisés dans la composition de l'invention présentent l'avantage de permettre la dispersion d'huiles de toute nature, aussi bien les huiles constituées de triglycérides, que les alcanes, les esters, les silicones, les filtres solaires, les perfluorées, soit seules soit en mélanges. En outre, ils présentent l'avantage d'être peu sensibles aux variations de pH pour des valeurs comprises entre 4 et 8, qui sont les valeurs habituelles des compositions cosmétiques.

La taille moyenne des gouttes d'huiles des dispersions obtenues est généralement comprise entre 100 nm et 10 µm.

La viscosité des dispersions obtenues peut aller de très fluide (spray) à très visqueuse (crème) et elle est ajustée en fonction de la teneur en polymère introduite, du taux de phase huileuse émulsionnée et du procédé d'émulsification utilisé selon que l'on veut obtenir une émulsion micronique (diamètre moyen des gouttes d'huile supérieur ou égal à 1 µm) ou une émulsion submicronique (diamètre moyen des gouttes d'huile inférieur ou égal à 1 µm). La composition de l'invention a donc une viscosité qui peut varier largement selon le but final recherché. Ainsi, sa viscosité peut aller par exemple de 0,01 Pa.s à 100 Pa.s à une température de 25°C, viscosité mesurée au Rhéomat 180 pour une vitesse de rotation du mobile de 200 RPM (tour par minute).

Les procédés d'émulsification peuvent être réalisés avec des appareils de type pâle ou hélice, rotor-stator, et aussi par homogénéisation à haute pression (HHP).
- Pour obtenir des émulsions stables avec un faible taux de polymère (ratio huile/ polymère >25), il est possible de faire la dispersion en phase concentrée puis de diluer la dispersion avec le reste de la phase aqueuse.
- Il est également possible, par HHP (entre 50 et 800 bars), d'obtenir des dispersions stables avec des tailles de gouttes pouvant descendre jusqu'à 100 nm.

### Polymères

Les polymères utilisés dans la composition de l'invention sont hydrosolubles ou hydrodispersibles. On entend par "polymère hydrosoluble ou hydrodispersible", un polymère qui, introduit dans de l'eau à une concentration égale à 1 % en poids, conduit à une solution macroscopiquement homogène dont la transmittance de la lumière, à une longueur d'onde égale à 500 nm, à travers un échantillon de 1 cm d'épaisseur, est d'au moins 10 %, ce qui correspond à une absorbance [abs = -log (transmittance)] inférieure à 1,5.

Ce sont des polymères amphiphiles, c'est-à-dire des polymères qui ont une chaîne hydrophile et qui comportent un ou plusieurs motifs hydrophobes.

Les polymères conformes à l'invention ont en général une masse molaire en poids allant de 50 000 à 10 000 000, plus préférentiellement de 100 000 à 8 000 000 et encore plus préférentiellement de 200 000 à 3 000 000.

Les polymères conformes à l'invention sont de façon préférentielle neutralisés partiellement ou totalement par une base minérale telle que, par exemple, soude, potasse, ammoniaque, ou par une base organique telle que la mono-, di- et triéthanolamine, l'aminométhylpropanediol, la N- méthyl-glucamine, les acides aminés basiques comme l'arginine et la lysine, et leurs mélanges.

Les polymères utilisés selon l'invention peuvent être obtenus selon les procédés classiques de polymérisation radicalaire en présence d'un ou plusieurs initiateurs tels que par exemple l'azobisisobutyronitrile (AIBN), l'azobisdiméthylvaléronitrile, le chlorhydrate de 2,2-azobis-[2-amidinopropane] (ABAH = 2,2-azoBis-[2-Amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂0₂ éventuellement en présence de réducteurs.

Les polymères sont notamment obtenus par polymérisation radicalaire en milieu tert-butanol dans lequel ils précipitent. En utilisant la polymérisation dans le tert-butanol, il est possible d'obtenir une distribution de la taille des particules de polymère particulièrement favorable pour ses utilisations.

La réaction de polymérisation peut être conduite à une température comprise entre 0°C et 150°C, de préférence entre 20°C et 100°C, soit à pression atmosphérique, soit sous pression réduite. Elle peut aussi être réalisée sous atmosphère inerte, et de préférence sous azote.

Le polymère utilisé selon l'invention est un polymère comprenant comme motif de formule (II), un motif où p =0, c'est-à-dire que le motif de formule (II) est exempt de groupes oxypropylénés et ne comporte que les motifs oxyéthylénés, et que ce motif a la formule (III) indiquée ci-dessous : 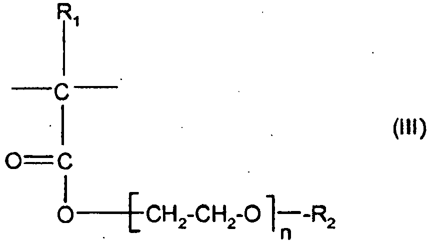 dans laquelle n désigne un nombre de moles et varie de 6 à 25 ; R₁ désigne un radical méthyle, et R₂ désigne un radical alkyle linéaire ou ramifié comportant de 16 à 25 atomes de carbone, et de préférence un radical alkyle linéaire.

Selon un mode préféré de réalisation de l'invention, dans la formule (II), p = 0 ; R₁ est le radical méthyle ; n est un nombre entier allant de 6 à 25 et R₂ est un radical alkyle linéaire en C₁₆-C₂₅.

On peut ainsi utiliser les polymères préparés à partir de l'acide 2-acrylamido-2-méthylpropane-sulfonique (AMPS) ou l'un de ses sels, notamment de sodium ou d'ammonium, avec un ester de l'acide acrylique ou d'acide méthacrylique et :
- d'un alcool en C₁₆-C₁₈ oxyéthyléné par 8 moles d'oxyde d'éthylène (Genapol T-080 de la société Clariant),
- d'un alcool en C₁₆-C₁₈ oxyéthyléné par 11 moles d'oxyde d'éthylène (Genapol T-110 de la société Clariant),
- d'un alcool en C₁₆-C₁₈ oxyéthyléné par 15 moles d'oxyde d'éthylène (Genapol T-150 de la société Clariant),
- d'un alcool en C₁₆-C₁₈ oxyéthyléné par 20 moles d'oxyde d'éthylène (Genapol T-200 de la société Clariant),
- d'un alcool en C₁₆-C₁₈ oxyéthyléné par 25 moles d'oxyde d'éthylène (Genapol T-250 de la société Clariant),
- d'un alcool en C₁₈-C₂₂ oxyéthyléné par 25 moles d'oxyde d'éthylène,
- d'un alcool en iso-C₁₆-C₁₈ oxyéthyléné par 25 moles d'oxyde d'éthylène.

Selon un mode préféré de réalisation, le polymère utilisé selon l'invention est un copolymère d'AMPS et de méthacrylate d'alcool en C₁₆-C₁₈ comportant de 6 à 25 moles d'oxyde d'éthylène, ce copolymère étant obtenu à partir d'AMPS et d'acide méthacrylique ou d'un sel d'acide méthacrylique et d'un alcool en C₁₆-C₁₈ oxyéthyléné comportant 6 à 25 moles d'oxyde d'éthylène.

Selon un mode encore plus préféré de réalisation de l'invention, le polymère utilisé selon l'invention est un copolymère d'AMPS et de méthacrylate d'alcool en C₁₆-C₁₈ comportant de 6 à 10 moles d'oxyde d'éthylène, dont la proportion molaire d'unités méthacrylate d'alcool en C₁₆-C₁₈ (motifs (II)) va de 2% à 15%, et encore mieux de 5% à 10%. Il s'agit plus particulièrement un copolymère d'AMPS et d'acide méthacrylique ou d'un sel d'acide méthacrylique et d'alcool stéarylique oxyéthyléné (Genapol T-080) comportant une proportion molaire d'unités méthacrylate d'alcool de 7,35 %.

On peut citer en particulier comme polymère préféré selon l'invention, le copolymère d'AMPS et de sel d'ammonium d'acide méthacrylique et d'alcool stéarylique oxyéthyléné comportant 8 moles d'oxyde d'éthylène (méthacrylate de Genapol T-080) et comportant une proportion molaire d'unités méthacrylate d'alcool de 7,35 %, commercialisé sous la dénomination Aristoflex SNC par la société Clariant (nom INCI: Ammonium Acryloyldimethyltaurate / Steareth-8 Methacrylate Copolymer)

La quantité de polymère(s) d'AMPS tel que défini ci-dessus est égale ou inférieure à 0,6 % en poids par rapport au poids total de la composition. La quantité (en matière active) de polymère(s) d'AMPS dans la composition de l'invention peut aller par exemple, en matière active, de 0,01 à 0,6 % en poids, de préférence de 0,05 à 0,5 % en poids, mieux de 0,1 à 0,5 % en poids par rapport au poids total de la composition.

Le rapport en poids de la quantité de polymère(s) (b) sur la quantité d'émulsionnant(s) (b) va de 0,7 à 1,3 et il va de préférence entre 0,9 à 1,1.

### Emulsionnants

La composition selon l'invention contient un ou plusieurs émulsionnants non ioniques ayant un point de fusion inférieur à 45°C. On entend par « ayant un point de fusion inférieur à 45°C », des émulsionnants non ioniques qui sont liquides à une température inférieure à 45°C.

La quantité (en matière active) d'émulsionnant(s) peut aller par exemple de 0,007 à 0,78 % en poids, de préférence de 0,035 à 0,65 % en poids, et mieux de 0,14 à 0,65% en poids par rapport au poids total de la composition.

Les émulsionnants sont choisis parmi les esters d'acide gras et de polyol y compris ceux comportant au moins un motif oxyéthyléné.

On entend par « acide gras » ou par « alcool gras », des acides ou des alcools ayant une chaîne alkyle, linéaire ou ramifiée, comportant de 8 à 30 atomes de carbone, et de préférence de 12 à 24 atomes de carbone.

Parmi les esters d'acide gras et de polyol, y compris ceux comportant au moins un motif oxyéthyléné, on peut citer notamment :
- les esters de glycéryle, qui peuvent être des mono-, di- ou de poly-alkylesters de glycéryle, de diglycéryle, ou de polyglycéryle, tels que le mono-isostéarate de glycéryle, tel que le produit commercialisé sous la dénomination de Peceol Isostearique (Nom INCI : Glyceryl Isostearate) par la société Gattefosse ; l'isostéarate polyglycérolé (4 moles) (nom INCl : Polyglyceryl-4 Isostearate) commercialisé sous la dénomination Isolan Gl34 par la société Goldschmidt, l'isostéarate de diglycéryle, vendu par la société SOLVAY; le laurate de glycérylel comportant 2 unités de glycérol, vendu par la société SOLVAY (nom INCl : Polyglyceryl-2 Laurate), le monoisostearate polyglycérolé (10 moles) vendu sous la dénomination Nikkol decaglyn 1-IS par la société Nihon Surfactant (Nom INCI: Polyglyceryl-10 isostearate), le diisostearate de polyglyceryl-2 vendu sous la dénomination Dermol DGDIS par la société Alzo (Nom INCI: Polyglyceryl-2-diisostearate).
- les esters de polyéthylène glycol (ou esters de PEG), qui sont des esters formés de 1 à 200 unités d'oxyde d'éthylène et d'au moins une chaîne d'acide gras comportant de 8 à 30, et de préférence de 12 à 22 atomes de carbone. La chaîne grasse des esters peut être notamment une chaîne isostéarate. A titre d'exemples d'esters de polyéthylène glycol, on peut citer les esters d'acide stéarique oxyéthylénés tels que le PEG 20 stéarate commercialisé sous la dénomination de Myrj 49 par la société Uniqema, les esters d'acide isostéarique oxyéthylénés tels que l'isostéarate de PEG-8 comme le produit commercialisé sous la dénomination Prisorine 3644 par la société Uniqema.

La composition selon l'invention peut contenir un ou plusieurs de ces émulsionnants.

Selon un mode préféré de réalisation de l'invention, l'émulsionnant est choisi parmi les esters de glycéryle, les esters de polyéthylène glycol, et leurs mélanges, et plus particulièrement parmi :
- l'isostéarate de glycéryle, commercialisé sous la dénomination Peceol Isostearique par la société Gattefosse ;
- les esters d'acide stéariques et de polyéthylène glycol tels que le PEG-20 stearate,

### Phase aqueuse

Avantageusement, le polymère amphiphile est introduit dans la phase aqueuse de l'émulsion. En outre, la phase aqueuse contient de l'eau et éventuellement un ou plusieurs composés miscibles à l'eau ou au moins en partie miscibles à l'eau, comme les polyols ; les mono-alcools inférieurs en C₂ à C₈, tels que l'éthanol et l'isopropanol. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple les glycols comme le butylène glycol, le propylène glycol, l'isoprène glycol, le glycérol et les polyéthylène glycols comme le PEG-8, le sorbitol, les sucres comme le glucose.

La phase aqueuse peut comprendre aussi tout additif habituel hydrosoluble ou hydrodispersible comme indiqué ci-après.

La phase aqueuse peut représenter de 60 à 98 % en poids, de préférence de 65 à 95 % en poids, mieux de 70 à 90 % en poids, et encore mieux de 70 à 85 % en poids par rapport au poids total de la composition.

Le ou les composés miscibles à l'eau, tels que polyols et alcools inférieurs, peuvent être présents en une quantité allant de 0 à 30 % du poids total de la composition notamment de 0,1 à 30 % et mieux en une quantité allant de 1 à 20 %.

### Phase huileuse

La nature de la phase huileuse de l'émulsion selon l'invention n'est pas critique. La phase huileuse est une phase grasse contenant au moins un corps gras choisi parmi les huiles liquides à température ambiante (20-25°C), volatiles ou non, d'origine végétale, minérale ou synthétique, et leurs mélanges. Ces huiles sont physiologiquement acceptables.

La phase huileuse peut comprendre aussi tout additif habituel liposoluble ou lipodispersible comme indiqué ci-après.

La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode préféré de réalisation, la composition de l'invention comporte au moins une huile choisie parmi les huiles de silicone, les hydrocarbures linéaires ou ramifiés, les éthers et les esters de synthèse, et leurs mélanges, et notamment choisie parmi les huiles de silicone volatiles et les hydrocarbures ramifiés comme l'huile de Parléam®, et leurs mélanges.

La quantité de phase huileuse dans la composition de l'invention est inférieure à 40 % du poids total de l'émulsion et elle peut aller par exemple de 2 à 40 % en poids, de préférence de 5 à 35 % en poids, mieux de 10 à 30 % en poids et encore mieux de 15 à 30 % en poids par rapport au poids total de la composition. Comme indiqué plus haut, cette quantité de phase huileuse ne comprend pas la quantité d'émulsionnant.

### Additifs

De façon connue, la composition pour application topique de l'invention peut contenir également un ou plusieurs des adjuvants habituels dans le domaine cosmétique ou dermatologique. Comme adjuvants, on peut citer par les gélifiants, les actifs, les conservateurs, les antioxydants, les parfums, les solvants, les sels, les charges, les filtres solaires (= filtres U.V.), les matières colorantes, les agents basiques (triéthanolamine, diéthanolamine, hydroxyde de sodium) ou acides (acide citrique), et encore les vésicules lipidiques ou tout autre type de vecteur (nanocapsules, microcapsules, etc...), et leurs mélanges. Ces adjuvants sont utilisés dans les proportions habituelles dans le domaine cosmétique, et par exemple de 0,01 à 30 % du poids total de la composition, et ils sont, selon leur nature, introduits dans la phase aqueuse de la composition ou dans la phase huileuse, ou encore dans des vésicules ou tout autre type de vecteur. Ces adjuvants ainsi que leurs concentrations doivent être tels qu'ils ne modifient pas la propriété recherchée pour l'émulsion de l'invention.

Selon la viscosité désirée pour la composition selon l'invention, on peut y incorporer un ou plusieurs gélifiants, hydrophiles ou lipophiles. Comme gélifiants hydrophiles, on peut citer par exemple les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Noveon ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme guar, les alginates, les celluloses modifiées ou non ; et leurs mélanges. Quand ils sont présents, ces gélifiants doivent être introduits en quantité telle qu'ils ne modifient pas les propriétés de la composition selon l'invention. Comme gélifiants lipophiles, on peut citer notamment les argiles modifiées telles que le silicate de magnésium modifié (bentone gel VS38 de RHEOX), l'hectorite modifiée par le chlorure de distéaryl diméthyl ammonium (nom INCI : Disteardimonium hectorite) commercialisé sous la dénomination « bentone 38 CE » par la société RHEOX.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphériques organiques, les fibres ; et leurs mélanges. Comme poudres sphériques organiques, on peut citer par exemple les poudres de polyamide et notamment les poudres de Nylon® telles que Nylon-1 ou Polyamide 12, commercialisées sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle commercialisées par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges. Ces charges peuvent être présentes dans des quantités allant de 0 à 20 % en poids et de préférence de 0,5 à 10 % en poids par rapport au poids total de la composition.

Comme actifs utilisables dans la composition de l'invention, on peut citer par exemple, les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine K, la vitamine C (acide ascorbique), la vitamine B5 (panthénol), la vitamine B3 ou PP (niacinamide), les dérivés de ces vitamines (notamment esters) et leurs mélanges ; les agents kératolytiques et/ou desquamants tels que l'acide salicylique et ses dérivés, les alpha-hydroxyacides comme l'acide lactique et l'acide glycolique et leurs dérivés, l'acide ascorbique et ses dérivés ; l'urée ; la caféine ; les dépigmentants tels que l'acide kojique, l'hydroquinone et l'acide caféique ; l'acide salicylique et ses dérivés ; les rétinoïdes tels que les caroténoïdes et les dérivés de vitamine A ; l'hydrocortisone ; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les enzymes ; les flavonoïdes ; les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les céramides ; les agents anti-inflammatoires ; les agents apaisants ; les agents matifiants ; les agents anti-chute et/ou repousse des cheveux ; les agents anti-rides ; les huiles essentielles ; et leurs mélanges ; et tout actif approprié pour le but final de la composition.

Les filtres U.V. peuvent être organiques ou minéraux (ou filtres U.V. physiques). Ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649.

On peut citer plus particulièrement les filtres UV suivant, désignés ci-dessous sous leur nom INCI :
Dérivés de l'acide para-aminobenzoique :
   - PABA,
   - Ethyl PABA,
   - Ethyl Dihydroxypropyl PABA,
   - Ethylhexyl Diméthyl PABA commercialisé notamment sous le nom ESCALOL 507 par la société ISP,
   - Glyceryl PABA,
   - PEG-25 PABA commercialisé sous le nom UVINUL P25 par la société BASF.
Dérivés salicyliques :
   - Homosalate commercialisé sous le nomEusolex HMS par la société Rona/EM Industries,
   - Ethylhexyl Salicylate commercialisé sous le nom NEO HELIOPAN OS par la société Haarmann et Reimer,
   - Dipropyleneglycol Salicylate commercialisé sous le nom DIPSALpar la société SCHER,
   - TEA Salicylate, commercialisé sous le nom NEO HELIOPAN TS par la société Haarmann et Reimer,
Dérivés du dibenzoylméthane :
   - Butyl Methoxydibenzoylmethane commercialisé notamment sous le nom commercial PARSOL 1789 par la société Hoffmann La Roche,
   - Isopropyl ibenzoylmethane,
Dérivés cinnamiques :
   - Ethylhexyl Methoxycinnamate commercialisé notamment sous le nom commercial PARSOL MCX par la société Hoffmann La Roche,
   - Isopropyl Methoxy cinnamate,
   - Isoamyl Methoxy cinnamate commercialisé sous le nom commercial NEO HELIOPAN E 1000 par la société Haarmann et Reimer,
   - Cinoxate,
   - DEA Methoxycinnamate,
   - Diisopropyl Methylcinnamate,
   - Glyceryl Ethylhexanoate Dimethoxycinnamate
Dérivés de β,β'-diphénylacrylate :
   - Octocrylene commercialisé notamment sous le nom commercial UVINUL N539 par la société BASF,
   - Etocrylene, commercialisé notamment sous le nom commercial UVINUL N35 par la société BASF,
Dérivés de la benzophénone :
   - Benzophenone-1 commercialisé sous le nom commercial UVINUL 400 par la société BASF,
   - Benzophenone-2 commercialisé sous le nom commercial UVINUL D50 par la société BASF
   - Benzophenone-3 ou Oxybenzone, commercialisé sous le nom commercial UVINUL M40 par la société BASF,
   - Benzophenone-4 commercialisé sous le nom commercial UVINUL MS40 par la société BASF,
   - Benzophenone-5,
   - Benzophenone-6 commercialisé sous le nom commercial Helisorb 11 par la société Norquay ;
   - Benzophenone-8 commercialisé sous le nom commercial Spectra-Sorb UV-24 par la société American Cyanamid ;
   - Benzophenone-9 commercialisé sous le nom commercial UVINUL DS-49 par la société BASF,
   - Benzophenone-12.
Dérivé du benzylidène camphre :
   - 3-Benzylidene camphor fabriqué sous le nom MEXORYL SD par la société Chimex,
   - 4-Methylbenzylidene camphor commercialisé sous le nom EUSOLEX 6300 par la société Merck ,
   - Benzylidene Camphor Sulfonic Acid fabriqué sous le nom MEXORYL SL par la société Chimex,
   - Camphor Benzalkonium Methosulfate fabriqué sous le nom MEXORYL SO par la société Chimex,
   - -Terephthalylidene Dicamphor Sulfonic Acid fabriqué sous le nom MEXORYL SX par la société Chimex,
   - Polyacrylamidomethyl Benzylidene Camphor fabriqué sous le nom MEXORYL SW par la société Chimex.
Dérivés du phenyl benzimidazole :
   - Phenylbenzimidazole Sulfonic Acid commercialisé notamment sous le nom commercial EUSOLEX 232 par la société Merck,
   - Benzimidazilate commercialisé sous le nom commercial NEO HELIOPAN AP par la société Haarmann et REIMER,
Dérivés de la triazine :
   - Anisotriazine commercialisé sous le nom commercial TINOSORB S par la société Ciba Geigy,
   - Ethylhexyl triazone commercialisé notamment sous le nom commercial UVINUL T150 par la société BASF,
   - Diethylhexyl Butamido Triazone commercialisé sous le nom commercial UVASORB HEB par la société SIGMA 3V.
Dérivés du phenyl benzotriazole :
   - Drometrizole Trisiloxane commercialisé sous le nom Silatrizole par la société Rhodia Chimie ,
   - Methylène bis-Benzotriazolyl Tetramethylbutylphénol, commercialisé sous forme solide sous le nom commercial MIXXIM BB/100 par la société Fairmount Chemical ou sous forme micronisé en dispersion aqueuse sous le nom commercial TINOSORB M par la société Ciba Specialty Chemicals.
Dérivés anthraniliques :
   - Menthyl anthranilate commercialisé sous le nom commercial NEO HELIOPAN MA par la société la société Haarmann et Reimer.
Dérivés d'imidazolines :
   - Ethylhexyl Dimethoxybenzylidene Dioxoimidazoline Propionate,
Dérivés du benzalmalonate :
   - Polyorganosiloxane à fonctions benzalmalonate commercialisé sous la dénomination commerciale PARSOL SLX par la société Hoffmann La Roche
   et les mélanges de ces filtres.

Les filtres UV organiques plus particulièrement préférés sont choisis parmi les composés suivants :
- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Benzimidazilate,
- Anisotriazine,
- Ethylhexyl triazone,
- Diethylhexyl Butamido Triazone,
- Methylene bis-Benzotriazolyl Tetramethylbutylphenol,
- Drometrizole Trisiloxane,
et leurs mélanges.

La quantité totale de filtres UV organiques dans les compositions selon l'invention peut aller par exemple de 0,1 à 20% en poids par rapport au poids total de la composition, et de préférence allant de 0,2 à 15% en poids par rapport au poids total de la composition.

Comme filtres physiques pouvant être ajoutés dans la composition de l'invention, on peut citer par exemple les pigments et nanopigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Les compositions de l'invention sont préparées selon les procédés habituels de préparation des émulsions H/E. Le copolymère amphiphile d'AMPS est solubilisé sous agitation dans la phase aqueuse, de préférence à température ambiante (25°C), et l'émulsion est préparée par introduction de la phase huileuse dans la phase aqueuse sous agitation.

Les compositions selon l'invention peuvent se présenter par exemple sous toutes les formes galéniques des émulsions H/E, par exemple sous forme de sérum, de lait ou de crème, et elles sont préparées selon les méthodes usuelles. Les compositions, objets de l'invention, sont destinées à une application topique et peuvent constituer notamment une composition dermatologique ou cosmétique, par exemple destinée au soin (anti-rides, anti-âge, hydratation, protection solaire, etc), au traitement, au nettoyage et au maquillage des matières kératiniques et notamment de la peau, des lèvres, des cheveux, des cils et des ongles des êtres humains.

Selon un mode préféré de réalisation de l'invention, la composition constitue une composition cosmétique et est destinée à une application topique sur la peau.

Aussi, l'invention a encore pour objet l'utilisation cosmétique d'une composition cosmétique telle que définie ci-dessus pour le soin, le démaquillage et/ou le nettoyage de la peau, des lèvres et/ou des cheveux.

L'invention a enfin pour objet un procédé de traitement cosmétique de la peau, y compris du cuir chevelu, des cheveux, et/ou des lèvres, caractérisé par le fait qu'on applique sur la peau, les cheveux et/ou les lèvres, une composition cosmétique telle que définie ci-dessus.

Les exemples qui suivent permettront de mieux comprendre l'invention, sans toutefois présenter un caractère limitatif. Les quantités indiquées sont en % en poids, sauf mention contraire.

### V) Exemples

### Mode général de préparation des émulsions:

Les copolymères amphiphiles de l'invention, fournis sous forme de poudre, sont solubilisés dans l'eau pendant 30 minutes sous agitation à 25°C ; la solution obtenue est macroscopiquement homogène. L'émulsion est préparée par introduction lente de la phase huileuse dans la phase aqueuse sous agitation à l'aide d'un homogénéisateur de type Moritz sous une vitesse d'agitation de 4000 RPM pendant :
- 15 minutes pour les émulsions comprenant des gouttes de taille supérieure à 1 µm,
- 5 minutes puis l'émulsion est affinée à l'aide d'un homogénéisateur de type Rannie sous une pression égale à 500 bars (3 passages) pour les émulsions comprenant des gouttes de taille inférieure à 1 µm.

La taille des gouttes des émulsions microniques est mesurée à l'aide d'un granulomètre laser Hydro 2000 S/G (Malvern) en considérant le diamètre moyen en surface D(3,2) et la taille des gouttes des émulsions submicroniques est mesurée à l'aide d'un analyseur de taille BI-90 (Brookhaven instrument).

### Exemple 1 selon l'invention : Fluide hydratant pour le corps

| *Phase A* : | |
|---|---|
| Eau distillée | 83,175 % |
| Conservateur | 1 % |
| Triéthanolamine | 0,002 % |
| Copolymère d'AMPS et de méthacrylate de Génapol T-080 (7,35% en mole) (Aristoflex SNC) | 0,4 % |
| | |

| *Phase B* : | |
|---|---|
| Huile de Parleam | 9 % |
| Cyclohexadiméthylsiloxane | 6 % |
| Isostéarate de glycéryle (Peceol isotearique de Gattefosse) | 0,4 % |

La phase B a été ajoutée sous très vive agitation à la phase A, à température ambiante. La taille des gouttes était de l'ordre de 3 µm. Sa viscosité mesurée au rhéomat 180 à 25°C à une vitesse de rotation du mobile 3 de 200 RPM était de 0,068 Pa.s. Cette émulsion était stable après 2 mois à 55°C.

Cet exemple montre que l'association d'un polymère de l'invention et d'isostéarate de glycéryle, dans un rapport massique de concentrations égal à 1, permet de stabiliser l'émulsion. La faible teneur en polymère et en émulsionnant permet d'obtenir une émulsion ayant une bonne innocuité vis-à-vis de la peau.

### Exemple 2 comparatif

| *Phase A* : | |
|---|---|
| Eau distillée | 83,585 % |
| Conservateur | 1 % |
| Triéthanolamine | 0,002 % |
| Copolymère d'AMPS et de méthacrylate de Génapol T-080 (7,35% en mole) (Aristoflex SNC) | 0,4 % |
| | |

| *Phase B* : | |
|---|---|
| Huile de Parleam | 9 % |
| Cyclohexadiméthylsiloxane | 6 % |

La phase B a été ajoutée sous très vive agitation à la phase A, à température ambiante. La taille moyenne des gouttes obtenues était égale à 8 µm. la viscosité mesurée au Rhéomat 180 à 25°C à une vitesse de rotation du mobile 2 de 200 RPM était égale à 0,061 Pa.s L'émulsion s'est déstabilisée à 55°C après 5 jours.

Cet exemple comparatif de l'exemple 1 montre que la présence d'un émulsionnant non ionique liquide est nécessaire à l'obtention d'une émulsion stable.

### Exemple 3 comparatif

| *Phase A* : | |
|---|---|
| Eau distillée | 83,385 % |
| Conservateur | 1 % |
| Triéthanolamine | 0,001 % |
| Copolymère d'AMPS et de méthacrylate de Génapol T-080 (7,35% en mole) (Aristoflex SNC) | 0,4 % |
| | |

| *Phase B* : | |
|---|---|
| Huile de Parleam | 9 % |
| Cyclohexadiméthylsiloxane | 6 % |
| Isostéarate de glycéryle (Peceol isotearique de Gattefosse) | 0,2 % |

La phase B a été ajoutée sous très vive agitation à la phase A, à température ambiante. La taille moyenne des gouttes obtenues était égale à 5 µm. La viscosité mesurée au rhéomat 180 à 25°C à une vitesse de rotation du mobile 2 de 200 RPM était égale à 0,060 Pa.s. L'émulsion est devenue grossière après 2 mois à 55°C, avec des globules de l'ordre de 15 µm.

Cet exemple comparatif à l'exemple 1 montre qu'un rapport en poids polymère/ émulsionnant égal à 2 n'est pas suffisant pour stabiliser l'émulsion.

### Exemple 4 comparatif :

| *Phase A* : | |
|---|---|
| Eau distillée | 82,975 % |
| Conservateur | 1 % |
| Triéthanolamine | 0,002 % |
| Copolymère d'AMPS et de méthacrylate de Genapol LA-070 (8,5% en mole) (Aristoflex LNC) | 0,5 % |
| | |

| *Phase B* : | |
|---|---|
| Huile de Parleam | 9 % |
| Cyclohexadiméthylsiloxane | 68 % |
| Isostéarate de glycéryle (Peceol isotearique de Gattefosse) | 0,5 % |

La phase B a été ajoutée sous vive agitation à la phase A. La taille des gouttes était de l'ordre de 5µm. Sa viscosité mesurée au rhéomat 180 à 25°C à une vitesse de rotation du mobile 2 de 200 RPM était de 0,060 Pa.s. L'émulsion est devenue grossière après deux mois à 55°C avec l'apparition de globules de taille 20 µm. Cet exemple comparatif montre que l'association d'un polymère ne faisant pas partie de l'invention où R2 dans la formule (II) comporte 12 à 14 atomes de carbone, tel que décrit dans la demande FR-2,853,527 avec un émulsionnant lipophile dans un rapport massique égal à 1 ne permet pas de stabiliser l'émulsion, pour une faible teneur en polymère émulsionnant (quantité de polymère ≤ 0,6%).

### Exemple 5 selon l'invention : Lait pour le corps

| *Phase A* : | |
|---|---|
| Eau distillée | 77,996 % |
| Conservateur | 1 % |
| Triéthanolamine | 0,004 % |
| Copolymère d'AMPS et de méthacrylate de Genapol T-080 (7,35% en mole) (Aristoflex SNC) | 0,5 % |
| | |

| *Phase B* : | |
|---|---|
| Huile de Parleam | 12 % |
| Cyclohexadiméthylsiloxane | 8 % |
| Isostéarate de glycéryle (Peceol isotearique de Gattefosse) | 0,5 % |

Mode opératoire : La phase B a été ajoutée sous vive agitation à la phase A .

On a obtenu un lait fluide, stable au moins 2 mois, et dont la taille des gouttes était de l'ordre de 2 µm. Sa viscosité mesurée au Rhéomat 180 à 25°C à une vitesse de rotation du mobile 2 de 200 RPM était de 0,123 Pa.s.

Cet exemple montre que l'association d'un polymère de l'invention et d'isostéarate de glycéryle dans le rapport défini selon l'invention permet d'obtenir une émulsion stable..

### Exemple 6 comparatif :

| *Phase A* : | |
|---|---|
| Eau distillée | 77,995 % |
| Conservateur | 1 % |
| Triéthanolamine | 0,005 % |
| Copolymère d'AMPS et de méthacrylate de Genapol T-080 (7,35% en mole) (Aristoflex SNC) | 0,5 % |
| | |

| *Phase B* : | |
|---|---|
| Huile de Parleam | 12 % |
| Cyclohexadiméthylsiloxane | 8 % |
| Acide stéarique | 0,5 % |

Mode opératoire : La phase B, préalablement chauffée à 65°C, a été ajoutée sous vive agitation à la phase A également à 65°C. On a obtenu un lait épais et dont la taille des gouttes était de l'ordre de 5 µm. Son pH était égal à 7,1 et sa viscosité mesurée au Rhéomat 180 à 25°C à une vitesse de rotation du mobile 3 de 200 RPM était de 0,069 Pa.s. Après 48 heures à 4°C, un phénomène de déstabilisation (coalescence) a été observé à l'échelle microscopique : la taille des gouttes a augmenté de 5 µm à 25 µm, ce qui montre une instabilité de l'émulsion.

Ce exemple comparatif montre qu'il est indispensable que l'émulsionnant soit non ionique pour obtenir une émulsion stable et qu'un émulsionnant ionique solide à 45°C tel que l'acide stéarique ne permet pas d'assurer la stabilité d'une émulsion huile dans eau à base des polymères de l'invention.

### Exemple 7 selon l'invention : Crème pour le visage

| *Phase A* : | |
|---|---|
| Eau distillée | 59,997 % |
| Conservateur | 1 % |
| Triéthanolamine | 0,003 % |
| Copolymère d'AMPS et de méthacrylate de Genapol T-080 (7,35% en mole) | 0,5 % |
| | |

| *Phase B* : | |
|---|---|
| Huile de Parleam | 22,8 % |
| Cyclohexadiméthylsiloxane | 15,2 % |
| Isostéarate de glycéryle (Peceol isotearique de Gattefosse) | 0,5 % |

Mode opératoire : La phase B a été ajoutée sous très vive agitation à la phase A, à température ambiante. On a obtenu une crème stable pendant au moins 2 mois ; la taille des gouttes était de l'ordre de 2µm. Sa viscosité mesurée au Rhéomat 180 à 25°C à une vitesse de rotation du mobile 3 de 200 RPM était de 0,21 Pa.s.

### Exemple 8 comparatif

| *Phase A* : | |
|---|---|
| Eau distillée | 78 % |
| Conservateur | 1 % |
| Copolymère d'AMPS et de méthacrylate de Génapol T-080 (7,35% en mole) (Aristoflex SNC) | 0,5 % |
| | |

| *Phase B* : | |
|---|---|
| Huile de Parleam | 12 % |
| Cyclohexadiméthylsiloxane | 8 % |
| Disodium stearoyl glutamate | 0,5 % |

Mode opératoire : La phase B, préalablement chauffée à 65°C, a été ajoutée sous vive agitation à la phase A également à 65°C. On a obtenu un lait épais et dont la taille des gouttes était de l'ordre de 5µm. Après 12 heures à 4°C, un phénomène de déstabilisation (crémage) a été observé à l'échelle macroscopique.

Ce exemple comparatif montre qu'il est nécessaire que l'émulsionnant utilisé soit non ionique pour obtenir une émulsion stable, et qu'un émulsionnant ionique tel que le disodium stéaroyl glutamate ne permet pas d'assurer la stabilité d'une émulsion huile dans eau à base des polymères de l'invention.

### Exemple 9 selon l'invention : Lait pour le corps

| *Phase A* : | |
|---|---|
| Eau distillée | 77,996 % |
| Conservateur | 1 % |
| Triéthanolamine | 0,004 % |
| Copolymère d'AMPS et de méthacrylate de Genapol T-080 (7,35% en mole) (Aristoflex SNC) | 0,5 % |
| | |

| *Phase B* : | |
|---|---|
| Huile de Parleam | 12 % |
| Cyclohexadiméthylsiloxane | 8 % |
| PEG 20 stéarate | 0,5 % |

Mode opératoire : La phase B a été ajoutée sous vive agitation à la phase A.

On a obtenu un lait fluide, stable pendant au moins 1 mois, et dont la taille des gouttes était de 3 µm. Sa viscosité mesurée au Rhéomat 180 à 25°C à une vitesse de rotation du mobile 2 de 200 RPM était de 0,096 Pa.s.

Cet exemple montre que l'association d'un polymère de l'invention et de PEG 20 stéarate (point de fusion égal à 28°C) permet de stabiliser l'émulsion.

### Exemple 10 comparatif

| *Phase A* : | |
|---|---|
| Eau distillée | 82,955 % |
| Conservateur | 1 % |
| Copolymère d'AMPS et de méthacrylate de Génapol T-080 (7,35% en mole) (Aristoflex SNC) | 1 % |
| | |

| *Phase B* : | |
|---|---|
| Huile de Parleam | 9 % |
| Cyclohexadiméthylsiloxane | 6 % |

Mode opératoire : La phase B a été ajoutée à la température ambiante sous vive agitation à la phase A. On a obtenu une crème gélifiée très élastique dont la taille des gouttes égale à 3 µm. Cette crème gélifiée n'était pas cosmétique du fait de son élasticité et de sa mauvaise prise au doigt.

Ce exemple comparatif montre qu'il est nécessaire de considérer une teneur en polymère émulsionant faible pour obtenir une texture agréable du point de vue cosmétique et facile à appliquer.

## Revendications

1. Composition pour application topique, sous forme d'émulsion huile-dans-eau comprenant une phase huileuse dispersée dans une phase aqueuse, **caractérisée en ce qu'**elle contient (a) un ou plusieurs émulsionnants non ioniques ayant un point de fusion inférieur à 45°Cchoisi parmi les esters d'acide gras et de polyol y compris ceux comportant au moins un motif oxyéthyléné, et leurs mélanges, et (b) un ou plusieurs polymères non réticulés comprenant de (A) 80 à 99 % en moles de motif acide 2-acrylamido 2-méthylpropane sulfonique de formule (I) : 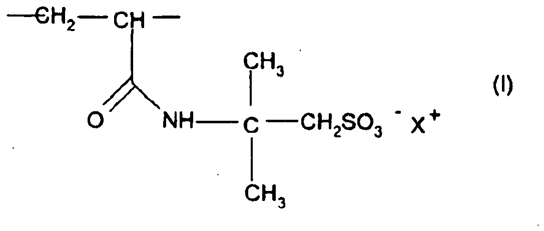 dans laquelle X⁺ est un proton, un cation de métal alcalin, un cation alcalino-terreux ou l'ion ammonium ;
et (B) 1 à 20 % en moles de motif de formule (II) suivante : 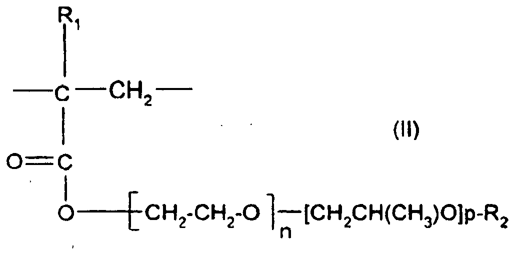 dans laquelle p = 0 ; R₁ est le radical méthyle ; n est un nombre entier allant de 6 à 25 et R₂ est un radical alkyle linéaire ou ramifié en C₁₆-C₂₅,
la quantité de polymère(s) (b) étant inférieure ou égale à 0,6 % en poids par rapport au poids total de la composition, et le rapport en poids entre la quantité de polymère(s) (b) et la quantité d'émulsionnant(s) (a) allant de 0,7 à 1,3.
et **en ce que** la phase huileuse est présente en une quantité inférieure à 40 % en poids par rapport au poids total de la composition.

2. Composition selon la revendication 1, **caractérisée en ce que** dans la formule (II), p = 0 ; R₁ est le radical méthyle ; n est un nombre entier allant de 6 à 25 et R₂ est un radical alkyle linéaire en C₁₈-C₂₅.

3. Composition selon la revendication 1 ou 2, **caractérisée en ce que** le polymère a une masse molaire en poids allant de 50 000 à 10 000 000.

4. Composition selon l'une quelconque des revendications précédentes, **caractérisée en ce que** le polymère est neutralisé partiellement ou totalement par une base minérale ou organique.

5. Composition selon l'une quelconque des revendications précédentes, **caractérisée en ce que** le polymère est préparé à partir de l'acide 2-acrylamido-2-méthylpropane-sulfonique (AMPS) ou l'un de ses sels de sodium ou d'ammonium, avec un ester de l'acide acrylique ou d'acide méthacrylique et :
- d'un alcool en C₁₆-C₁₉ oxyéthyléné par 8 moles d'oxyde d'éthylène,
- d'un alcool en C₁₆-C₁₈ oxyéthyléné par 11 moles d'oxyde d'éthylène,
- d'un alcool en C₁₆-C₁₈ oxyéthyléné par 15 moles d'oxyde d'éthylène,
- d'un alcool en C₁₆-C₁₈ oxyéthyléné par 20 moles d'oxyde d'éthylène,
- d'un alcool en C₁₆-C₁₈ oxyéthyléné par 25 moles d'oxyde d'éthylène,
- d'un alcool en C₁₈-C₂₂ oxyéthyléné par 25 moles d'oxyde d'éthylène,
- d'un alcool en iso-C₁₆-C₁₈ oxyéthyléné par 25 moles d'oxyde d'éthylène.

6. Composition selon l'une quelconque des revendications précédentes, **caractérisée en ce que** la quantité de polymère va de 0,01 à 0,6 % en poids par rapport au poids total de la composition.

7. Composition selon l'une quelconque des revendications précédentes, **caractérisée en ce que** la quantité d'émulsionnant va de 0,007 à 0,78 % en poids, de préférence de 0,035 à 0,65% en poids, par rapport au poids total de la composition.

8. Composition selon la revendication précédente, **caractérisée en ce que** l'émulsionnant est choisi parmi les esters de glycéryle, les esters de polyéthylène glycol, et leurs mélanges.

9. Composition selon l'une quelconque des revendications précédentes, **caractérisée en ce qu'**elle constitue une composition cosmétique.

10. Utilisation cosmétique d'une composition cosmétique selon l'une quelconque des revendications 1 à 8, pour le soin, le démaquillage et/ou le nettoyage de la peau, des lèvres et/ou des cheveux.

11. Procédé de traitement cosmétique de la peau, des cheveux, et/ou des lèvres, **caractérisé en ce qu'**on applique sur la peau, les cheveux et/ou les lèvres, une composition cosmétique selon l'une quelconque des revendications 1 à 8.

## Patentansprüche

1. Zuaammensetzung für topische Anwendung in Form einer Öl-in-Wasser-Emulsion, die eine in einer wässrigen Phase dispergierte Ölphase umfasst, **dadurch gekennzeichnet, dass** sie Folgendes enthält: (a) einen oder mehrere nicht-ionische Emulgatoren mit einem Schmelzpunkt unter 45 °C, die aus den Estern von Fettsäure und Polyol einschließlich derjenigen, die mindestens eine Oxyethyleneinheit aufweisen, und deren Gemischen ausgewählt sind, und (b) ein oder mehrere nicht vernetzte Polymere umfassend (A) 80 bis 99 Mol-% einer 2-Acrylamido-2-methylpropansulfonsäureeinheit der Formel (I): 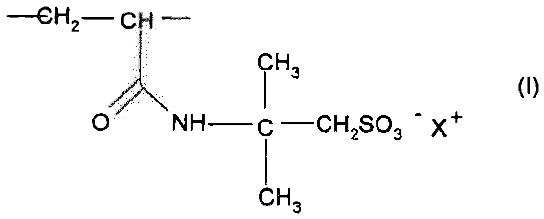 wobei X⁺ ein Proton, ein Alkalimetallkation, ein Erdalkalimetallkation oder das Ammoniumion ist;
und (B) 1 bis 20 Mol-% einer Einheit der folgenden Formel (II): 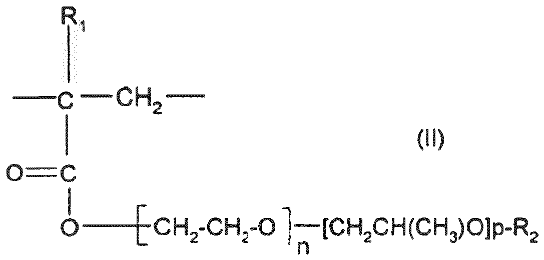 wobei p = 0 ist; R₁ das Methylradikal ist; n eine ganze Zahl von 6 bis 25 ist und R₂ ein lineares oder verzweigtes C₁₆-C₂₅-Alkylradikal ist,
wobei die Menge an Polymer(en) (b) kleiner oder gleich 0,6 Gew.-% beträgt, bezogen auf das Gesamtgewicht der Zusammensetzung, und das Gewichtsverhältnis zwischen der Menge an Polymer(en) (b) und der Menge an Emulgator(en) (a) 0,7 bis 1,3 beträgt,
und dadurch, dass die Ölphase in einer Menge von weniger als 40 Gew.-% vorhanden ist, bezogen auf das Gesamtgewicht der Zusammensetzung.

2. Zusammensetzung nach Anspruch 1, **dadurch gekennzeichnet, dass** in der Formel (II) p = 0 ist; _{R}1 das Methylradikal ist; n eine ganze Zahl von 6 bis 25 ist und R₂ ein lineares C₁₆-C₂₅-Alkylradikal ist.

3. Zusammensetzung nach Anspruch 1 oder 2, **dadurch gekennzeichnet, dass** das Polymer ein Gewichtsmittel des Molekulargewichts von 50 000 bis 10 000 000 aufweist.

4. Zusammensetzung nach einem der vorhergehenden Ansprüche, **dadurch gekennzeichnet, dass** das Polymer teilweise oder ganz durch eine mineralische oder organische Base neutralisiert wird.

5. Zuaammensetzung nach einem der vorhergehenden Ansprüche, **dadurch gekennzeichnet, dass** das Polymer ausgehend von 2-Acrylamido-2-methylpropansulfonsäure (AMPS) oder einem ihrer Natrium- oder Ammoniumsalze hergestellt wird, mit einem Acrylsäureester oder Methacrylsäureester und:
- einem C₁₆-C₁₈-Alkohol, oxyethyleniert mit 8 Mol Ethylenoxid,
- einem C₁₆-C₁₈-Alkohol, oxyethyleniert mit 11 Mol Ethylenoxid,
- einem C₁₆-C₁₈-Alkohol, oxyethyleniert mit 15 Mol Ethylenoxid,
- einem C₁₆-C₁₈-Alkohol, oxyethyleniert mit 20 Mol Ethylenoxid,
- einem C₁₆-C₁₈-Alkohol, oxyethyleniert mit 25 Mol Ethylenoxid,
- einem C₁₈-C₂₂-Alkohol, oxyethyleniert mit 25 Mol Ethylenoxid,
- einem C₁₆-C₁₈-Isoalkohol, oxyethyleniert mit 25 Mol Ethylenoxid.

6. Zusammensetzung nach einem der vorhergehenden Ansprüche, **dadurch gekennzeichnet, dass** die Menge an Polymer 0,01 bis 0,6 Gew.-% beträgt, bezogen auf das Gesamtgewicht der Zusammensetzung.

7. Zusammensetzung nach einem der vorhergehenden Ansprüche, **dadurch gekennzeichnet, dass** die Menge an Emulgator 0,007 bis 0,78 Gew.-%, vorzugsweise 0,035 bis 0,65 Gew.-% beträgt, bezogen auf das Gesamtgewicht der Zusammensetzung.

8. Zusammensetzung nach dem vorhergehenden Anspruch, **dadurch gekennzeichnet, dass** der Emulgator aus den Glycerylestern, den Polyethylenglykolestern und deren Mischungen ausgewählt ist.

9. Zusammensetzung nach einem der vorhergehenden Ansprüche, **dadurch gekennzeichnet, dass** sie eine kosmetische Zusammensetzung darstellt.

10. Kosmetische Verwendung einer kosmetischen Zusammensetzung nach einem der Ansprüche 1 bis 8 für die Pflege, das Abschminken und/oder die Reinigung der Haut, der Lippen und/oder der Haare.

11. Verfahren zur kosmetischen Behandlung der Haut, der Haare und/oder der Lippen, **dadurch gekennzeichnet, dass** auf die Haut, die Haare und/oder die Lippen eine kosmetische Zusammensetzung nach einem der Ansprüche 1 bis 8 aufgetragen wird.

## Claims

1. Composition for topical application, in the form of an oil-in-water emulsion comprising an oily phase dispersed in an aqueous phase, **characterized in that** it contains (a) one or more nonionic emulsifiers with a melting point of less than 45°C chosen from fatty acid esters of polyols, including those comprising at least one oxyethylene unit, and mixtures thereof, and (b) one or more non-crosslinked polymers comprising (A) 80 mol% to 99 mol% of 2-acrylamido-2-methylpropanesulfonic acid units of formula (I): 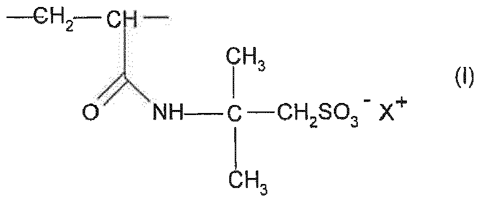 in which X⁺ is a proton, an alkali metal cation, an alkaline-earth metal cation or an ammonium ion;
and (B) 1 mol% to 20 mol% of units of formula (II) below: 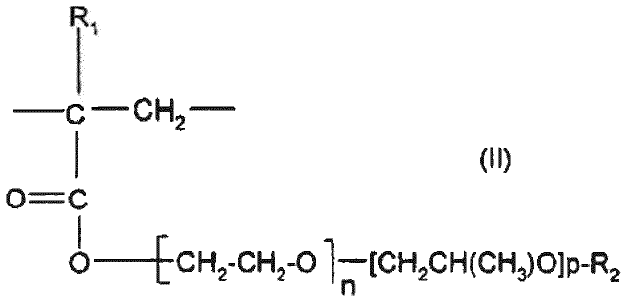 in which p = 0; R₁ is a methyl radical; n is an integer ranging from 6 to 25 and R₂ is a C₁₆-C₂₅ linear or branched alkyl radical,
the amount of polymer(s) (b) being less than or equal to 0.6% by weight relative to the total weight of the composition, and the weight ratio between the amount of polymer(s) (b) and the amount of emulsifier(s) (a) ranging from 0.7 to 1.3,
and **in that** the oily phase is present in an amount of less than 40% by weight relative to the total weight of the composition.

2. Composition according to Claim 1, **characterized in that**, in formula (II), p = 0; R₁ is a methyl radical; n is an integer ranging from 6 to 25 and R₂ is a C₁₆-C₂₅ linear alkyl radical.

3. Composition according to Claim 1 or 2, **characterized in that** the polymer has a weight-average molar mass ranging from 50 000 to 10 000 000.

4. Composition according to any one of the preceding claims, **characterized in that** the polymer is partially or totally neutralized with a mineral or organic base.

5. Composition according to any one of the preceding claims, **characterized in that** the polymer is prepared from 2-acrylamido-2-methylpropanesulfonic acid (AMPS) or a sodium or ammonium salt thereof, with an ester of acrylic or methacrylic acid and:
- of a C₁₆-C₁₈ alcohol oxyethylenated with 8 mol of ethylene oxide,
- of a C₁₆-C₁₈ alcohol oxyethylenated with 11 mol of ethylene oxide,
- of a C₁₆-C₁₈ alcohol oxyethylenated with 15 mol of ethylene oxide,
- of a C₁₆-C₁₈ alcohol oxyethylenated with 20 mol of ethylene oxide,
- of a C₁₆-C₁₈ alcohol oxyethylenated with 25 mol of ethylene oxide,
- of a C₁₈-C₂₂ alcohol oxyethylenated with 25 mol of ethylene oxide,
- of a C₁₆-C₁₈ isoalcohol oxyethylenated with 25 mol of ethylene oxide.

6. Composition according to any one of the preceding claims, **characterized in that** the amount of polymer ranges from 0.01% to 0.6% by weight relative to the total weight of the composition.

7. Composition according to any one of the preceding claims, **characterized in that** the amount of emulsifier ranges from 0.007% to 0.78% by weight and preferably from 0.035% to 0.65% by weight relative to the total weight of the composition.

8. Composition according to the preceding claim, **characterized in that** the emulsifier is chosen from glyceryl esters and polyethylene glycol esters, and mixtures thereof.

9. Composition according to any one of the preceding claims, **characterized in that** it constitutes a cosmetic composition.

10. Cosmetic use of a cosmetic composition according to any one of Claims 1 to 8, for caring for, removing makeup from and/or cleansing the skin, the lips and/or the hair.

11. Cosmetic process for treating the skin, the hair and/or the lips, **characterized in that** a cosmetic composition according to any one of Claims 1 to 8 is applied to the skin, the hair and/or the lips.
